# EUROPEAN PATENT SPECIFICATION

(11) **EP 3 941 289 B1**
(45) Date of publication and mention of the grant of the patent: **24.04.2024**
(21) Application number: 20721425.5
(22) Date of filing: 17.03.2020
(51) Int. Cl.: A24F 40/44, A61M 11/04, A61M 15/06, A24F 40/10, A24F 40/485, A61M 11/00

(54) **AEROSOL DELIVERY SYSTEM**
AEROSOLABGABESYSTEM
SYSTÈME D'ADMINISTRATION D'AÉROSOL

(30) Priority: 21.03.2019 EP 19164466
(43) Date of publication of application: 26.01.2022
(73) Proprietor: Imperial Tobacco Limited, Bristol, BS3 2LL (GB)
(72) Inventor: LORD, Chris, Liverpool Merseyside L24 9HP (GB); SUDLOW, Thomas, Liverpool Merseyside L24 9HP (GB); ILLIDGE, Ben, Liverpool Merseyside L24 9HP (GB); MADDEN, Alfred, Liverpool Merseyside L24 9HP (GB); ASTBURY, Ben, Liverpool Merseyside L24 9HP (GB)
(74) Representative: Mewburn Ellis LLP
(86) International application number: PCT/EP2020/057316
(87) International publication number: WO 2020/187922

(56) References cited:
- EP-A1- 3 311 683
- WO-A1-2016/154792
- WO-A1-2018/001106
- WO-A1-2018/058883
- WO-A1-2018/197511
- CN-U- 204 232 300
- CN-U- 206 079 033
- CN-U- 207 613 199
- CN-U- 207 707 306
- US-A1- 2015 272 218
- US-A1- 2016 143 365
- US-A1- 2017 006 916
- US-A1- 2017 049 154

## Description

### Field of the Invention

The present invention relates to an aerosol delivery system. The present invention preferably relates to an aerosol delivery system including a heater configured to heat an aerosol precursor to generate an aerosolised composition for inhalation by a user.

### Background

Pharmaceutical medicament, physiologically active substances and flavourings for example may be delivered to the human body by inhalation through the mouth and/or nose. Such material or substances may be delivered directly to the mucosa or mucous membrane lining the nasal and oral passages and/or the pulmonary system. For example, nicotine is consumed for therapeutic or recreational purposes and may be delivered to the body in a number of ways. Nicotine replacement therapies are aimed at people who wish to stop smoking and overcome their dependence on nicotine. Nicotine is delivered to the body in the form of aerosol delivery devices and systems, also known as smoking-substitute devices or nicotine delivery devices. Such devices may be non-powered or powered.

Devices or systems that are non-powered may comprise nicotine replacement therapy devices such as "inhalators", e.g. Nicorette^{®} Inhalator. These generally have the appearance of a plastic cigarette and are used by people who crave the behaviour associated with consumption of combustible tobacco - the so-called hand-to-mouth aspect - of smoking tobacco. Inhalators generally allow nicotine-containing aerosol to be inhaled through an elongate tube in which a container containing a nicotine carrier, for example, a substrate, is located. An air stream caused by suction through the tube by the user carries nicotine vapours into the lungs of the user to satisfy a nicotine craving. The container may comprise a replaceable cartridge, which includes a cartridge housing and a passageway in the housing in which a nicotine reservoir is located. The reservoir holds a measured amount of nicotine in the form of the nicotine carrier. The measured amount of nicotine is an amount suitable for delivering a specific number of "doses". The form of the nicotine carrier is such as to allow nicotine vapour to be released into a fluid stream passing around or through the reservoir. This process is known as aerosolization and or atomization. Aerosolization is the process or act of converting a physical substance into the form of particles small and light enough to be carried on the air i.e. into an aerosol. Atomization is the process or act of separating or reducing a physical substance into fine particles and may include the generation of aerosols. The passageway generally has an opening at each end for communication with the exterior of the housing and for allowing the fluid stream through the passageway. A nicotine-impermeable barrier seals the reservoir from atmosphere. The barrier includes passageway barrier portions for sealing the passageway on both sides of the reservoir. These barrier portions are frangible so as to be penetrable for opening the passageway to atmosphere.

A device or a system that is powered can fall into two sub-categories. In both subcategories, such devices or systems may comprise electronic devices or systems that permit a user to simulate the act of smoking by producing an aerosol mist or vapour that is drawn into the lungs through the mouth and then exhaled. The electronic devices or systems typically cause the vaporization of a liquid containing nicotine and entrainment of the vapour into an airstream. Vaporization of an element or compound is a phase transition from the liquid phase to vapour i.e. evaporation or boiling. In use, the user experiences a similar satisfaction and physical sensation to those experienced from a traditional smoking or tobacco product, and exhales an aerosol mist or vapour of similar appearance to the smoke exhaled when using such traditional smoking or tobacco products.

A person of ordinary skill in the art will appreciate that devices or systems of the second, powered category as used herein include, but are not limited to, electronic nicotine delivery systems, electronic cigarettes, e-cigarettes, e-cigs, vaping cigarettes, pipes, cigars, cigarillos, vaporizers and devices of a similar nature that function to produce an aerosol mist or vapour that is inhaled by a user. Such nicotine delivery devices or systems of the second category incorporate a liquid reservoir element generally including a vaporizer or misting element such as a heating element or other suitable element, and are known, inter alia, as atomizers, cartomizers, or clearomizers. Some electronic cigarettes are disposable; others are reusable, with replaceable and refillable parts.

Aerosol delivery devices or systems in a first sub-category of the second, powered category generally use heat and/or ultrasonic agitation to vaporize a solution comprising nicotine and/or other flavouring, propylene glycol and/or glycerine-based base into an aerosol mist of vapour for inhalation.

Aerosol delivery devices or systems in a second sub-category of the second, powered category may typically comprise devices or systems in which tobacco is heated rather than combusted. During use, volatile compounds may be released from the tobacco by heat transfer from the heat source and entrained in air drawn through the aerosol delivery device or system. Direct contact between a heat source of the aerosol delivery device or system and the tobacco heats the tobacco to form an aerosol. As the aerosol containing the released compounds passes through the device, it cools and condenses to form an aerosol for inhalation by the user. In such devices or systems, heating, as opposed to burning, the tobacco may reduce the odour that can arise through combustion and pyrolytic degradation of tobacco.

Aerosol delivery devices or systems falling into the first sub-category of powered devices or systems may typically comprise a powered unit, comprising a heater element, which is arranged to heat a portion of a carrier that holds an aerosol precursor. The carrier comprises a substrate formed of a "wicking" material, which can absorb aerosol precursor liquid from a reservoir and hold the aerosol precursor liquid. Upon activation of the heater element, aerosol precursor liquid in the portion of the carrier in the vicinity of the heater element is vaporised and released from the carrier into an airstream flowing around the heater and carrier. Released aerosol precursor is entrained into the airstream to be borne by the airstream to an outlet of the device or system, from where it can be inhaled by a user.

The heater element is typically a resistive coil heater, which is wrapped around a portion of the carrier and is usually located in the liquid reservoir of the device or system. Consequently, the surface of the heater may always be in contact with the aerosol precursor liquid, and long-term exposure may result in the degradation of either or both of the liquid and heater. Furthermore, residues may build up upon the surface of the heater element, which may result in undesirable toxicants being inhaled by the user. Furthermore, as the level of liquid in the reservoir diminishes through use, regions of the heater element may become exposed and overheat.

The present invention has been devised in light of the above considerations.

CN 206 079 033 U discloses an aerosol delivery system according to the preamble of claim 1. Other aerosol delivery systems are known from US 2016/143365 A1, EP 3 311 683 A1, US 2015/272218 A1, WO 2018/001106 A1, WO 2016/154792 A1, CN 207 707 306 U, CN 207 613 199 U, US 2017/006916 A1, WO 2018/058883 A1 and CN 204 232 300 U.

### Summary of the Invention

According to the invention, there is provided an aerosol delivery system according to claim 1. The dependent claims define preferred embodiments of the invention.

The present invention proposes that an aerosol generation apparatus has a fluid-transfer article with a first region which holds an aerosol precursor, the first region being arranged to transfer the aerosol precursor to a second region of the fluid-transfer article. That second region has two parts of different materials, one part being adjacent to the first region and the second part being of a material resistant to higher temperatures than the material of the first part. The first part has a plurality of holes therein and the second part extends across those holes so that aerosol precursor in the holes will pass to the second part of the second region. The second part is porous for passage therethrough of the aerosol precursor from the holes to an activation surface.

The aerosol-generation apparatus also has a heater, which heater contacts the activation surface so as to interact thermally therewith. The heater is not bonded to the activation surface, instead it makes abutting unbonded contact so that the heater and the activation surface are separable.

The separability of the fluid-transfer article and the heater means that it is possible to replace the fluid-transfer article without having to replace the heater. Since the aerosol precursor will be consumed when the apparatus is used by a user, it will normally be necessary to replace or at least refill the fluid-transfer article periodically, as it acts as a reservoir for the aerosol precursor.

The two different materials of the second region of the fluid-transfer article allow one (the material of the second part) to be adapted to the heater, whilst the other (the material of the first part) may be a lower cost material.

As mentioned above the first part of the second region has a plurality of holes therein. Those holes do not act as capillaries, but instead are of a size or sizes so that they cooperate with the second part of the second region to define non-capillary spaces in the second region in to which the aerosol precursor is able to flow. Thus, the aerosol precursor is able to pass from the first region in a non-capillary manner into the holes, and impinge on the second part of the second region. It is then able to pass through the second part due to the porous nature of the second part.

The heater is mounted in contact with the activation surface of the second region. In such an arrangement, there will normally be an air-flow pathway adjacent at least part of the activation surface, so that vapour or aerosol/vapour mixture released from the activation surface by the heating effect of the heater may mix with the air-flow and pass to the user. Furthermore, in such an arrangement, the air-flow pathway will normally pass on the opposite side of the heater from the activation surface.

The second region of the fluid-transfer article thus acts as a wick, to cause aerosol precursor to move from the first region to the activation surface where it may be heated by the heater. The wick has a two-layer structure, formed by the two parts of the second region. One of those parts is preferably being made of an inexpensive material through which the holes pass, and the second part, according to the invention as claimed, is of a more heat resistant material, which will interact with the heater at the activation surface. Aerosol precursor will be drawn through the second region, partly because the holes will fill with aerosol precursor, and partly because of the porous nature of the second part of the second region.

The heater is preferably a coil, mesh or foil. There may then be an air-flow pathway adjacent at least a part of the activation surface. Since the heater is in contact with the activation surface, a part of said air-flow pathway may be on the opposite of the heater from the activation surface.

Preferably, said first part of said second region is formed of a solid polymer material having said plurality of holes therein.

It is usually preferable that said second part of said second region is formed of fibrous material. That fibrous material may be ceramic fibre, glass fibre or carbon fibre. Alternatively, the second part of the second region may be porous glass or porous ceramic. Another possibility is that the second part of the second region is of a porous polymer material. Another possibility is for the first region of the fluid-transfer article to be a simple reservoir filled with liquid aerosol precursor, from which reservoir the liquid flows into the holes in the first part of the second region of the fluid-transfer article.

Preferably, the plurality of holes are moulded holes. As mentioned above, it is desirable that the first part of the second region is formed of solid polymer material and it is convenient to mould the holes at the same time that the first part itself is moulded.

The fluid-transfer article may act as a reservoir for aerosol precursor. One option is for the first region of said fluid-transfer article to be of porous polymer material.

The porous polymer material of the first region may comprise Polyetherimide (PEI) and/or Polyether ether ketone (PEEK) and/or Polytetrafluoroethylene (PTFE) and/or Polyimide (PI) and/or Polyethersulphone (PES) and/or Ultra-High Molecular Weight Polyethylene (UHMWPE) and/or Polypropylene (PP) and/or Polyethylene Terephthalate (PET). Similar materials may be used for the second part of the second region when that second region is made of a porous polymer material, as mentioned above.

Alternatively, the first region of the fluid-transfer article may be a simple hollow reservoir which is filled with aerosol precursor when the apparatus is to be used.

The aerosol-generation apparatus forms part of an aerosol delivery system which has a carrier which includes a first housing containing the fluid-transfer article. The aerosol delivery system then includes a further housing supporting the heater. The first housing and the further housing are mutually separable, to allow the carrier to be removed from the rest of the aerosol delivery system.

The further housing may have an inlet with the air-flow pathway extending to the inlet. It may also have a plate mounted in the further housing at a position spaced from the heater so that the air-flow pathway passes between the activation surface and the plate. The plate may optionally have a plurality of recesses in its surface facing the activation surface with the air-flow pathway passing through said recesses.

The invention includes the combination of the aspects and preferred features described except where such a combination is clearly impermissible or expressly avoided, as long as the resulting subject-matter falls within the scope of the claims.

### Summary of the Figures

So that the invention may be understood, and so that further aspects and features thereof may be appreciated, embodiments illustrating the principles of the invention will now be discussed in further detail with reference to the accompanying figures, in which:
**Figure 1** is a perspective view illustration of a system for aerosol delivery according to one or more embodiments of the present invention;
**Figure 2** is a cross-section side view illustration of part of an apparatus of the system for aerosol delivery of Figure 1;
**Figure 3** is a cross-section side view illustration of the system and apparatus for aerosol delivery of Figure 1;
**Figure 4** is a perspective view illustration of an aerosol carrier for use in the system for aerosol delivery according to one or more embodiments of the present invention;
**Figure 5** is a cross-section side view of elements of an aerosol carrier and a part of an apparatus of the system for aerosol delivery according to one or more embodiments of the present invention;
**Figure 6** is a cross-section side view of elements of an aerosol carrier and a part of an apparatus of the system for aerosol delivery according to one or more embodiments of the present invention, in an alternative configuration from that of Figure 5;
**Figure 7** is a cross-section side view of aerosol carrier according to one or more embodiments of the present invention;
**Figure 8** is a perspective cross-section side view of the aerosol carrier of Figure 7, and;
**Figure 9** is an exploded perspective view illustration of a kit-of-parts for assembling the system according to one or more embodiments of the present invention.

### Detailed Description of the Invention

Aspects and embodiments of the present invention will now be discussed with reference to the accompanying figures. Further aspects and embodiments will be apparent to those skilled in the art.

In general outline, one or more embodiments in accordance with the present invention may provide a system for aerosol delivery in which an aerosol carrier may be inserted into a receptacle (e.g. a "heating chamber") of an apparatus for initiating and maintaining release of an aerosol from the aerosol carrier. Another end, or another end portion, of the aerosol carrier may protrude from the apparatus and can be inserted into the mouth of a user for the inhalation of aerosol released from the aerosol carrier cartridge during operation of the apparatus.

Hereinafter, and for convenience only, "system for aerosol delivery" shall be referred to as "aerosol delivery system".

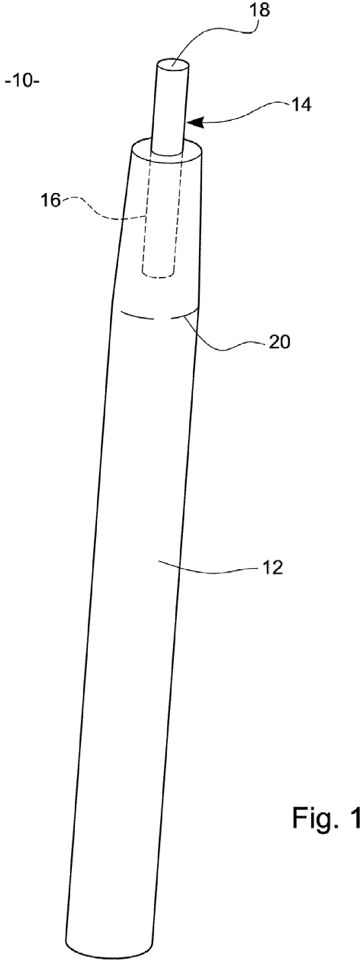

Referring now to Figure 1, there is illustrated a perspective view of an aerosol delivery system 10 comprising an aerosol generation apparatus 12 operative to initiate and maintain release of aerosol from a fluid-transfer article in an aerosol carrier 14. In the arrangement of Figure 1, the aerosol carrier 14 is shown with a first end 16 thereof and a portion of the length of the aerosol carrier 14 located within a receptacle of the apparatus 12. A remaining portion of the aerosol carrier 14 extends out of the receptacle. This remaining portion of the aerosol carrier 14, terminating at a second end 18 of the aerosol carrier, is configured for insertion into a user's mouth. A vapour and/or aerosol is produced when a heater (not shown in Figure 1) of the apparatus 12 heats a fluid-transfer article in the aerosol carrier 14 to release a vapour and/or an aerosol, and this can be delivered to the user, when the user sucks or inhales, via a fluid passage in communication with an outlet of the aerosol carrier 14 from the fluid-transfer article to the second end 18.

The device 12 also comprises air-intake apertures 20 in the housing of the apparatus 12 to provide a passage for air to be drawn into the interior of the apparatus 12 (when the user sucks or inhales) for delivery to the first end 16 of the aerosol carrier 14, so that the air can be drawn across an activation surface of a fluid-transfer article located within a housing of the aerosol carrier cartridge 14 during use. Optionally, these apertures may be perforations in the housing of the apparatus 12.

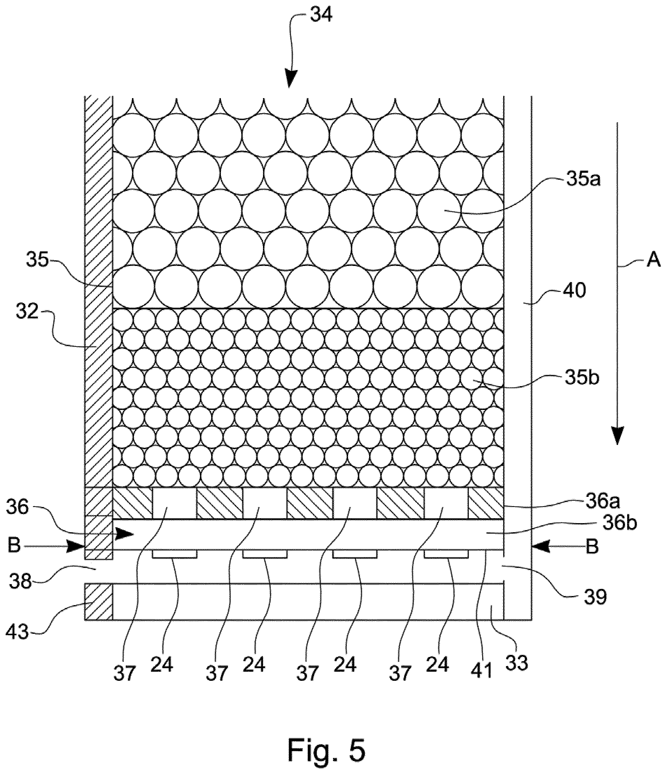

A fluid-transfer article 34 (not shown in Figure 1, but described hereinafter with reference to Figs. 5 to 8) is located within a housing of the aerosol carrier 14. The fluid-transfer article 34 contains an aerosol precursor material, which may include at least one of: nicotine; a nicotine precursor material; a nicotine compound; and one or more flavourings. The fluid-transfer article 34 is located within the housing of the aerosol carrier 14 to allow air drawn into the aerosol carrier 14 at, or proximal, the first end 16, and has first and second regions, as will be described.

The first region of the fluid-transfer article 34 may comprise a substrate of porous material where pores of the porous material hold, contain, carry, or bear the aerosol precursor material. In particular, the porous material of the fluid-transfer article may be a porous polymer material such as, for example, a sintered material. Particular examples of material suitable for the fluid-transfer article include: Polyetherimide (PEI); Polytetrafluoroethylene (PTFE); Polyether ether ketone (PEEK); Polyimide (PI); Polyethersulphone (PES); and Ultra-High Molecular Weight Polyethylene. Other suitable materials may comprise, for example, BioVyonTM (by Porvair Filtration Group Ltd) and materials available from Porex^{®}. Further optionally, a substrate forming the fluid-transfer article may comprise Polypropylene (PP) or Polyethylene Terephthalate (PET). All such materials may be described as heat resistant polymeric wicking material in the context of the present invention.

Alternatively, in some embodiments it is envisaged that the first region of the fluid-transfer article 34 may take the form of a simple tank having a cavity defining a hollow reservoir to hold the aerosol precursor.

The aerosol carrier 14 is removable from the apparatus 12 so that it may be disposed of when expired. After removal of a used aerosol carrier 14, a replacement aerosol carrier 14 can be inserted into the apparatus 12 to replace the used aerosol carrier 14.

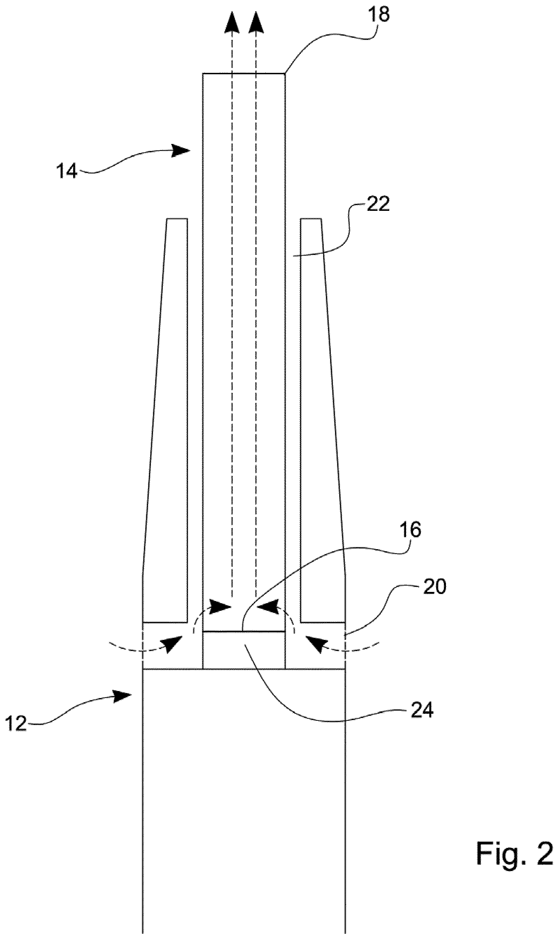

Figure 2 is a cross-sectional side view illustration of a part of apparatus 12 of the aerosol delivery system 10. The apparatus 12 comprises a receptacle 22 in which is located a portion of the aerosol carrier 14. In one or more optional arrangements, the receptacle 22 may enclose the aerosol carrier 14. The apparatus 12 also comprises a heater 24, which is in contact with an activation surface of the fluid-transfer article 34 when an aerosol carrier 14 is located within the receptacle 22. Optional configurations of the heater 24 will be discussed later.

Air flows into the apparatus 12 (in particular, into a closed end of the receptacle 22) via air-intake apertures 20. From the closed end of the receptacle 22, the air is drawn into the aerosol carrier 14 (under the action of the user inhaling or sucking on the second end 18) and expelled at the second end 18. As the air flows into the aerosol carrier 14, it passes across the activation surface. Heat from the heater 24, which is in contact with the activation surface of the fluid-transfer article 34, causes vaporisation of aerosol precursor material at the activation surface of the fluid-transfer article 34 and an aerosol is created in the air flowing over the activation surface. Thus, through the application of heat to the activation surface, an aerosol is released, or liberated, from the fluid-transfer article, and is drawn from the material of the aerosol carrier unit by the air flowing across the activation surface and is transported in the air flow to via outlet conduits (not shown in Figure 2) in the housing of the aerosol carrier 14 to the second end 18. The direction of air flow is illustrated by arrows in Figure 2.

To achieve release of the captive aerosol from the fluid-transfer article, the activation surface of the fluid-transfer article 34 is heated by the heater 24. As a user sucks or inhales on second end 18 of the aerosol carrier 14, the aerosol released from the fluid-transfer article and entrained in the air flowing across the activation surface is drawn through the outlet conduits (not shown) in the housing of the aerosol carrier 14 towards the second end 18 and onwards into the user's mouth.

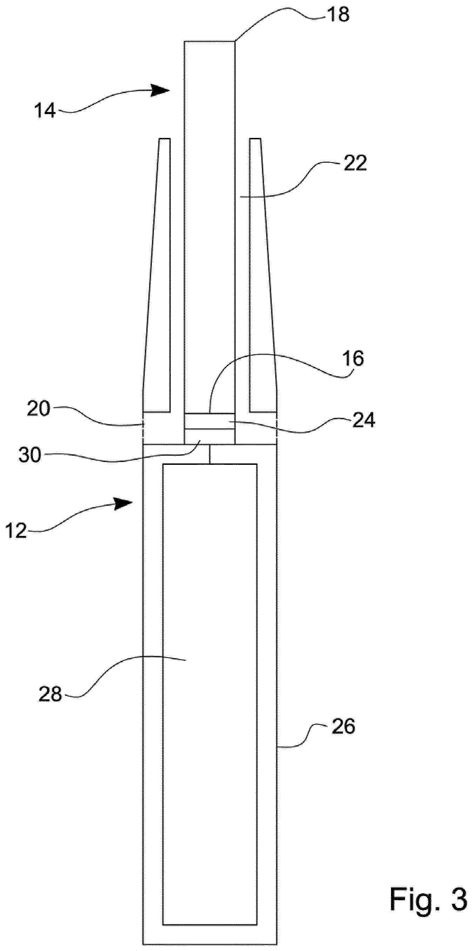

Turning now to Figure 3, a cross-sectional side view of the aerosol delivery system 10 is schematically illustrated showing the features described above in relation to Figures 1 and 2 in more detail. As can be seen, apparatus 12 comprises a housing 26, in which is located the receptacle 22. The housing 26 also contains control circuitry (not shown) operative by a user, or upon detection of air and/or vapour being drawn into the device 12 through air-intake apertures 20, i.e. when the user sucks or inhales. Additionally, the housing 26 comprises an electrical energy supply 28, for example a battery. Optionally, the battery comprises a rechargeable lithium ion battery. The housing 26 also comprises a coupling 30 for electrically (and optionally mechanically) coupling the electrical energy supply 28 to control circuitry (not shown) for powering and controlling operation of the heater 24.

Responsive to activation of the control circuitry of apparatus 12, the heater 24 heats the activation surface of the fluid-transfer article 34 (not shown in Figure 3). This heating process initiates (and, through continued operation, maintains) release of vapour and/or an aerosol from the activation surface of the fluid-transfer article 34. The vapour and/or aerosol formed as a result of the heating process is entrained into a stream of air being drawn across the activation surface of the fluid-transfer article 34 (as the user sucks or inhales). The stream of air with the entrained vapour and/or aerosol passes through the aerosol carrier 14 via outlet conduits (not shown) and exits the aerosol carrier 14 at second end 18 for delivery to the user. This process is briefly described above in relation to Figure 2, where arrows schematically denote the flow of the air stream into the device 12 and through the aerosol carrier 14, and the flow of the air stream with the entrained vapour and/or aerosol through the aerosol carrier cartridge 14.

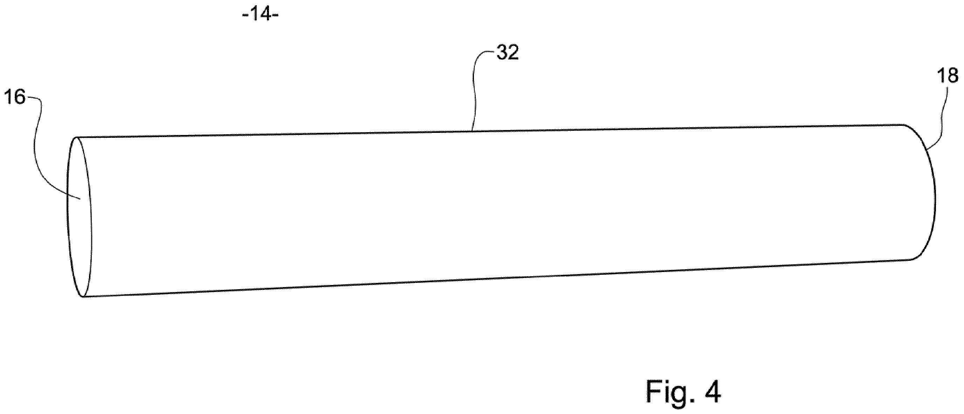

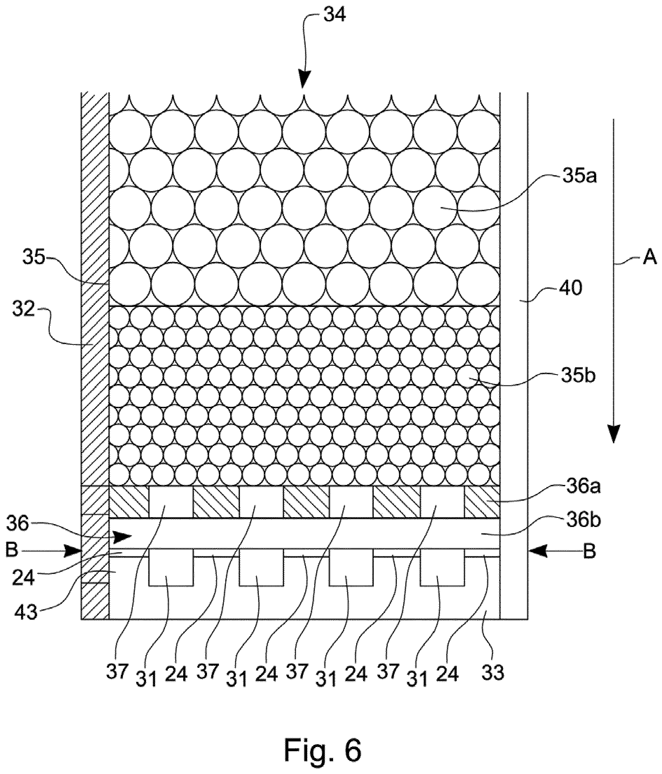

Figures 4 to 6 schematically illustrate the aerosol carrier 14 in more detail (and, in Figures 5 and 6, features within the receptacle in more detail). Figure 4 illustrates an exterior of the aerosol carrier 14, Figure 5 illustrates internal components of the aerosol carrier 14 in one optional configuration, and Fig. 6 illustrates internal components of the aerosol carrier 14 in another optional configuration.

Fig. 4 illustrates the exterior of the aerosol carrier 14, which comprises housing 32 for housing said fluid-transfer article (not shown). The particular housing 32 illustrated in Figure 4 comprises a tubular member, which may be generally cylindrical in form, and which is configured to be received within the receptacle of the apparatus. First end 16 of the aerosol carrier 14 is for location to oppose the heater of the apparatus, and second end 18 (and the region adjacent the second end 18) is configured for insertion into a user's mouth.

Figure 5 illustrates some internal components of the aerosol carrier 14 and of the heater 24 of apparatus 12, in one embodiment of the invention.

As described above, the aerosol carrier 14 comprises a fluid-transfer element 34. At least part of the fluid-transfer article 34 may be removable from the housing 32, to enable it to be replaced. The fluid-transfer article 34 acts as a reservoir for aerosol precursor and that aerosol precursor will be consumed as the apparatus is used. Once sufficient aerosol precursor has been consumed, the aerosol precursor will need to be replaced. It may then be easiest to replace it by replacing the fluid-transfer article 34, rather than trying to re-fill the fluid-transfer article 34 with aerosol precursor while it is in the housing 32.

In the illustrated embodiments, the fluid-transfer article 34 has a first region 35 formed by layers 35a and 35b, and a second region 36. That second region 36 has a first part being an upper layer 36a which is formed by a plate with a plurality of holes 37 therein, and a second part being a lower layer formed by a second plate 36b made of a porous material which allows aerosol precursor to pass therethrough. In the arrangement of Figure 5, the plate 36a with holes 37 therein is in contact with the first region 35 of the fluid-transfer article 34, so that aerosol precursor may pass from that first region 35 directly into the holes 37, and through those holes to the second plate 36b.

Since the second plate 36b is porous, the aerosol precursor will pass to the surface of the plate 36b remote from the first region 35 of the fluid-transfer article 34, which surface acts as an activation surface 41 of the fluid-transfer article 34. One or more heaters 24 are mounted on the activation surface 41. When the heater or heaters 24 are activated, the heat which they generate will be transferred to the activation surface 41.

Further components not shown in Figure 5 comprise: an inlet conduit, via which air can be drawn into the aerosol carrier 14; an outlet conduit, via which an air stream entrained with aerosol can be drawn from the aerosol carrier 14; a filter element; and a reservoir for storing aerosol precursor material and for providing the aerosol precursor material to the fluid-transfer article 34.

In Figure 5, the aerosol carrier is shown as comprising the fluid-transfer article 34 located within housing 32. The fluid transfer article 34 comprises a first region 35 holding an aerosol precursor. In one or more arrangements, the first region of 35 of the fluid transfer article 34 comprises a reservoir for holding the aerosol precursor. The first region 35 can be the sole reservoir of the aerosol carrier 14, or it can be arranged in fluid communication with a separate reservoir, where aerosol precursor is stored for supply to the first region 35. As shown in Figure 5, the first region 35 has a first layer 35a and a second layer 35b. The material forming the first layer 35a of the first region 35 comprises a porous structure, whose pore diameter size varies between one end of the first layer 35a and another end of the first layer 35a. The pore diameter size may increase from a first end remote from heater or heaters 24 (the upper end is as shown in the figure) to a second end. The pore diameter size may change in a step-wise manner (i.e. a first part with pores having a diameter of first size, and a second part with pores having a diameter of second, smaller size), or the change in pore size in the first layer 35a may be gradual rather than step-wise. This configuration of pores having a decreasing diameter size can provide a wicking effect, which can serve to draw fluid through the first layer 35a, towards heater or heaters 24.

The first region 35 of the fluid transfer article 34 may also comprise a second layer 35b. Aerosol precursor is drawn from the first layer 35a to the second layer 35b by the wicking effect of the material forming the first layer 35a. Thus, the first layer 35a is configured to transfer the aerosol precursor to the second layer 35b of the first region 35 of the fluid-transfer article 34.

The second layer 35b itself may comprise a porous structure formed by a porous polymer material. It is then preferable that the pore diameter size of the porous structure of the second layer 35b is smaller than the pore diameter size of the immediately adjacent part of the first layer 35a. As mentioned above, the porous polymer material may be a sintered material. Particular examples of material suitable for the fluid-transfer article include: Polyetherimide (PEI); Polytetrafluoroethylene (PTFE); Polyether ether ketone (PEEK); Polyimide (PI); Polyethersulphone (PES); and Ultra-High Molecular Weight Polyethylene. Other suitable materials may comprise, for example, BioVyonTM (by Porvair Filtration Group Ltd) and materials available from Porex^{®}. Further optionally, a substrate forming the fluid-transfer article may comprise Polypropylene (PP) or Polyethylene Terephthalate (PET).

However, as mentioned previously, in some embodiments it is envisaged that the first region 35 of the fluid-transfer article need not be of porous polymer material as described above. Instead, the first region 35 of the fluid-transfer article 34 may take the form of a simple tank having a cavity defining a hollow reservoir to hold the aerosol precursor. In such embodiments it is proposed that the plate 36a with holes 37 therein will extend across the bottom of the tank so that aerosol precursor held in the tank will impinge directly on the plate 36a and pass directly from the tank defining the first region 35 of the fluid-transfer article 34 into the holes 37 of the second region 36 of the fluid-transfer article.

As discussed above, the heater or heaters 24 transfer heat to the activation surface 41, thereby releasing aerosol precursor which has reached that activation surface 41 from the porous polymer material (or hollow reservoir) of the first region 35, through the second region 36. That vapour and/or a mixture of vapour and aerosol, may then pass into the air adjacent the activation surface 41 and the heater or heaters 24.

Figure 5 also illustrates an opening 38, which opening 38 is in communication with the air-intake apertures 20. A further opening 39 communicates with a duct 40 within the housing 32, which duct 40 communicates with the second end 18.

There is thus a fluid-flow path for air (referred to as an air-flow pathway) between openings 38 and 39, linking the apertures 20 and the second end 18 of the aerosol carrier. When the user sucks or inhales, air is drawn along the air-flow pathway, along the activation surface 41. A plate 33 forms a lower surface of the air-flow pathway, the plate 33 being spaced from the activation surface 41. It can be seen that the air-flow pathway is in direct contact with parts of the activation surface 41, as the heater or heaters 24 may partially block that path from the activation surface to the fluid flow pathway. The fluid flow pathway is on the opposite side of the heater or heaters 24 from the activation surface 41, so vapour must pass around the heater or heaters 24 if it cannot pass therethrough.

One or more droplets of the aerosol precursor will be released from the second plate 36b and heated, to release vapour or a mixture of aerosol and vapour into the air flowing in the air-flow pathway between the openings 38, 39. The vapour or mixture passes, as the user sucks and inhales, to the second end 18.

As mentioned above, the second region 36 of the fluid-transfer article 34 comprises a first plate 36a and a second plate 36b. The first plate 36a may be a moulded polymer disc so that is then easy to form the holes 37 therein by moulding the holes 37 when the plate 36a is itself moulded. The holes 37 are sufficiently large that they do not act as a capillaries, but instead define non-capillary spaces in the second region 36. Hence, aerosol precursor is able to pass from the first region 35 of the fluid-transfer article to the second region 36 in a non-capillary manner, into the holes 37, and then pass through the second plate 36b to the heater or heaters 24.

The second plate 36b is made of a porous material which is more heat-resistant than the material of the plate 36a, as it is acted on directly by the heater or heaters 24. It may be fibrous, made from e.g. ceramic fibre, glass fibre or carbon fibre. Alternatively, it may be formed from a high-temperature porous material such as porous glass or porous ceramic. Another possibility is that the second plate 36b may be of a porous polymer material, such as the materials described previously with reference to the layers 35a and 35b of the first region 35, provided that the polymer material is sufficiently resistant to the high temperatures to which it will be subject due to the heater or heaters 24.

It is thought that the flow of air between openings 38 and 39 along the activation surface 41 and past the heater or heaters 24 will have the effect of creating the lower air pressure adjacent the activation surface 41 which will tend to draw liquid through the porous second plate 36b to the activation surface 41. Thus, the transfer of aerosol precursor from the fluid-transfer article 34 is facilitated.

As mentioned above, the fluid-transfer article 34, formed by the first and second regions 35 and 36 and any further reservoir of aerosol precursor, forms the consumable part of the apparatus, in the sense that it can readily be replaced to enable the aerosol precursor to be replaced once it is consumed. The heater or heaters 24 are not part of the consumable elements. Thus, the housing 32 containing the fluid-transfer article 34 is separable from a housing 43 supporting the heater or heaters 24 along the line B-B in Figure 5. The plate 33 may be integral with the further housing 43, and the openings 38 and 39 are formed in the further housing 43. The further housing 43 may be integral with the housing 26 containing the electrical energy supply 28. It is for this reason that the heater or heaters 24 make contact with, but are not bonded to, the activation surface 41. The contact ensures the most efficient heat transfer from the heater or heaters 24 to the second plate 36b to heat the activation surface 41 but the heater or heaters 24 must be separable from that activation surface 41 to allow removal of the housing 32 from the further housing 43 when the fluid-transfer article 34 has become depleted. The line B to B may therefore correspond to the plane of the activation surface 41.

In Figure 5, the heater or heaters 24 may be separate or be interconnected to form a single heater. For example, the heater may be a coil, mesh or foil heater in which the parts of the heater 24 illustrated in Figure 5 may be parts of a common structure. Such a coil, mesh or foil heater is preferred so that any restriction caused by the heater or heaters 24 on release of aerosol or vapour from the activation surface is minimised, as vapour and/or aerosol may pass through the heater or heaters 24. However, it is also possible for the heater or heaters 24 to be a solid unbroken strip or strips, provided that there is then enough of the activation surface 41 not covered by the heater or heaters 24 to allow sufficient release of vapour and/or aerosol from the activation surface 41.

In the illustrative examples of Figure 5, the first layer 35a of the first region 35 of the fluid-transfer article 34 is located at an "upstream" end of the fluid-transfer article 34 and the second plate 35b of the second region 35b is located at a downstream" end of the fluid-transfer article 34. That is, aerosol precursor is wicked, or is drawn, from the "upstream" end of the fluid-transfer article 34 to the "downstream" end of the fluid-transfer article 34 (as denoted by arrow A in Figure 5).

In the arrangement of Figure 5, the plate 33 has a planar surface facing the activation surface 41. Figure 6 illustrates an arrangement in which the plate 33 has projections and recesses in its upper surface, so that the recesses can form channels 31 for air to flow therethrough. Other features which are the same as those of Figure 5 are indicated by the same reference numerals. Thus, the channels 31 form the air-flow pathway along the activation surface 41. In Figure 6, the projections and recesses form a square-wave or "castellated" structure. Further shapes are possible, however, such as alternating peaks and troughs or recesses with curved walls. All such arrangements permit channels 31 to be formed and allow air to flow along the activation surface 41. This control of air flow improves the mixing of the vapourised aerosol precursor into the air flow.

In the embodiment of Figure 6, the peaks in the upper surface of the plate 33 extend to the heater or heaters 24, with the recesses between those peaks which form the channels 31 then being aligned with the holes 37 formed in the second plate 35b of the fluid-transfer article 34. Other alignments are possible, and the projections need not reach all the way to the heater or heaters 24. In general, however, the heater or heaters 24 may restrict release of the vapourised aerosol precursor from parts of the activation surface 41 on which those heater or heaters 24 are formed, so it will normally be desirable that the channels 31 are aligned with the part or parts of the activation surface 41 other than the part or parts on which the heater or heaters 24 are formed.

Note also that, in Figure 6, the openings 38 and 39 are not visible since they will be at the ends of the channels 31 to allow air to pass from the opening 38 in to the channels 31, and from those channels 31 out of the opening 39. Also, as in Figure 5, the housing 32 containing the fluid-transfer article 34 may be separable from the housing 43 containing the intermediate structure 36 and the heater or heaters 24 along the line B-B in Figure 6.

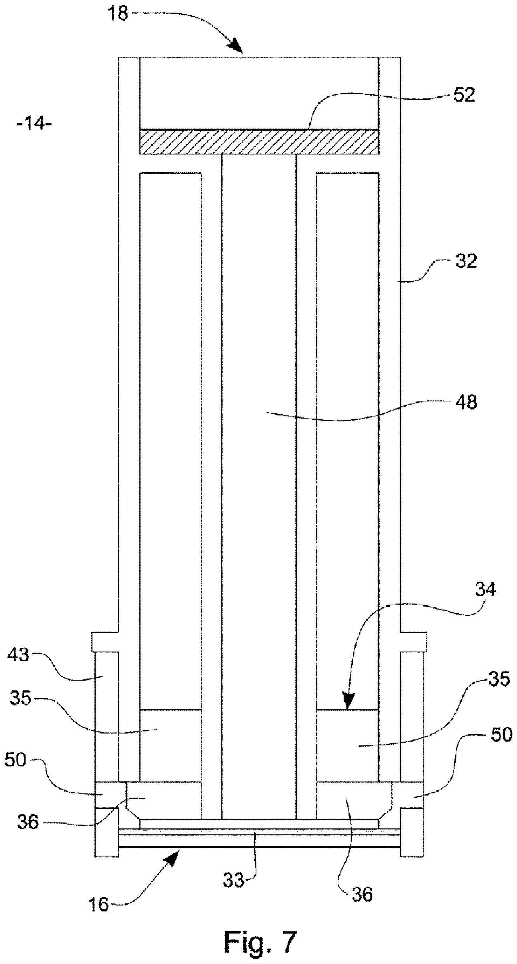

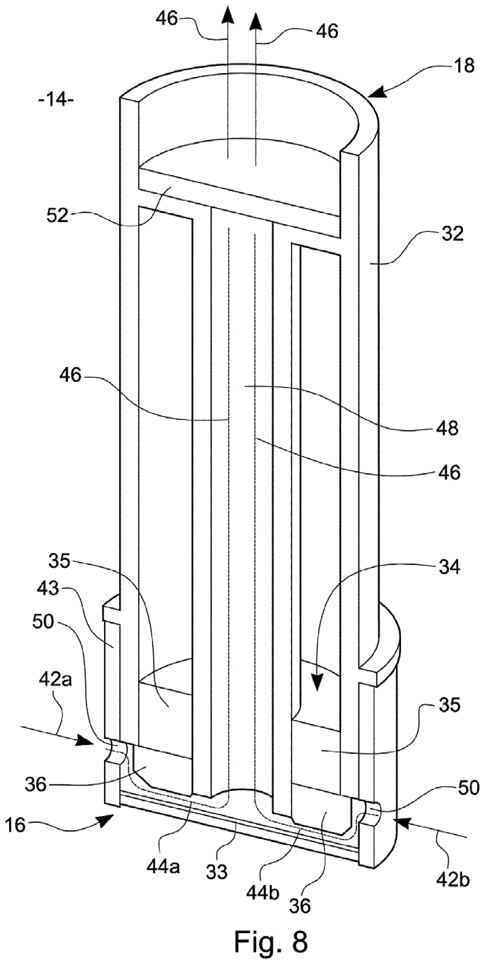

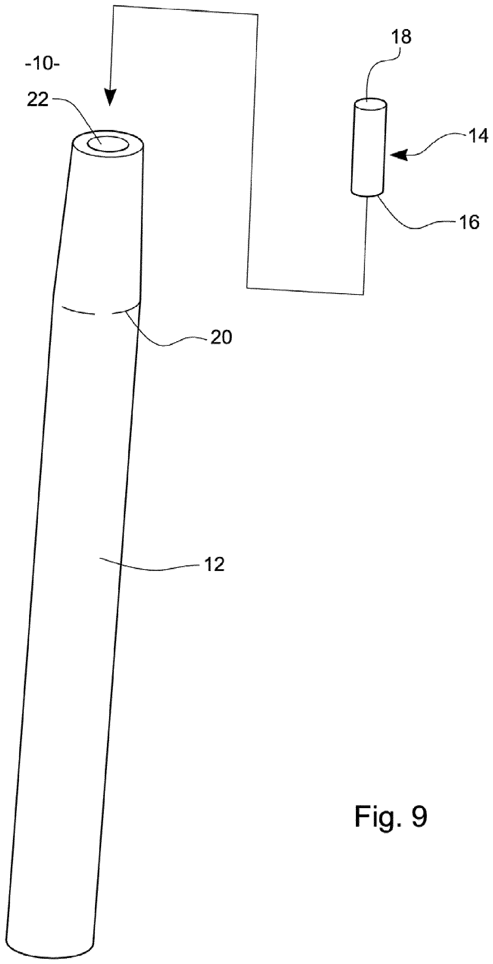

In the arrangements shown in Figures 5 and 6, the apertures 38, 39 are on opposite sides of the housing 32. Figures 7 and 8 shows an alternative configuration, in which the fluid-transfer article is annular, and both the first region 35 and the second region 36 are then in the form of annuli. In Figures 8 and 9, the structure of the fluid-transfer article 34, including the first region 35 and the second region 36 may correspond generally to that shown in Figure 5. The internal structure of the first and second regions 35 and 36 may be the same as in Figure 5, but are not illustrated in detail in Figures 7 and 8 for simplicity. The heater or heaters 24 also cannot be seen in Figures 7 and 8, but may be formed as in the arrangement of Figure 5 or Figure 6. However, the air flow in the apparatus is discussed in more detail below. Thus, Figures 7 and 8 illustrate an aerosol carrier 14 according to one or more possible arrangements in more detail. Figure 7 is a cross-section side view illustration of the aerosol carrier 14 and Figure 8 is a perspective cross-section side view illustration of the aerosol carrier 14.

As can be seen from Figures 7 and 8, the aerosol carrier 14 is generally tubular in form. The aerosol carrier 14 comprises housing 32, which defines the external walls of the aerosol carrier 14 and which defines therein a chamber in which are disposed the fluid-transfer article 34 (adjacent the first end 16 of the aerosol carrier 14) and internal walls defining the fluid communication pathway 48. Fluid communication pathway 48 defines a fluid pathway for an outgoing air stream from the channels 40 to the second end 18 of the aerosol carrier 14. In the examples illustrated in Figures 7 and 8, the fluid-transfer article 34 is an annular shaped element located around the fluid communication pathway 48. The housing 32 containing the fluid-transfer article 34 is separable from the housing 43 supporting heater or heaters 24.

In walls of the housing 43, there are provided inlet apertures 50 to provide a fluid communication pathway for an incoming air stream to reach the activation surface 41 of the second region 36 of the fluid-transfer article 34.

In the illustrated example of Figures 7 and 8, the aerosol carrier 14 further comprises a filter element 52. The filter element 52 is located across the fluid communication pathway 48 such that an outgoing air stream passing through the fluid communication pathway 48 passes through the filter element 52.

With reference to Figure 8, when a user sucks on a mouthpiece of the apparatus (or on the second end 18 of the aerosol carrier 14, if configured as a mouthpiece), air is drawn into the carrier through inlet apertures 50 extending through walls in the housing 32 of the aerosol carrier 14.

An incoming air stream 42a from a first side of the aerosol carrier 14 is directed to a first side of the second region 36 (e.g. via a gas communication pathway within the housing of the carrier). An incoming air stream 42b from a second side of the aerosol carrier 14 is directed to a second side of the second region 36 (e.g. via a gas communication pathway within the housing of the carrier). When the incoming air stream 42a from the first side of the aerosol carrier 14 reaches the first side of the second region 36, the incoming air stream 42a from the first side of the aerosol carrier 14 flows along the activation surface 41 of the second region 36. Likewise, when the incoming air stream 42b from the second side of the aerosol carrier 14 reaches the second side of the second region 36, the incoming air stream 42b from the second side of the aerosol carrier 14 flows along the activation surface 41 of the second region 36. The air streams from each side are denoted by dashed lines 44a and 44b in Figure 8. As these air streams 44a and 44b flow, aerosol precursor on the activation surface 41 of the second region 36 is entrained in air streams 44a and 44b.

In use, the heater or heaters 24 of the apparatus 12 raise a temperature of the second plate 36b of the second region 36 to a sufficient temperature to release, or liberate, captive substances (i.e. the aerosol precursor) to form a vapour and/or aerosol, which is drawn downstream. As the air streams 44a and 44b continue their passages, more released aerosol precursor is entrained within the air streams 44a and 44b. When the air streams 44a and 44b entrained with aerosol precursor meet at a mouth of the outlet fluid communication pathway 48, they enter the outlet fluid communication pathway 48 and continue until they pass through filter element 52 and exit outlet fluid communication pathway 48, either as a single outgoing air stream, or as separate outgoing air streams 46 (as shown). The outgoing air streams 46 are directed to an outlet, from where it can be inhaled by the user directly (if the second end 18 of the aerosol capsule 14 is configured as a mouthpiece), or via a mouthpiece. The outgoing air streams 46 entrained with aerosol precursor are directed to the outlet (e.g. via a gas communication pathway within the housing of the carrier).

Figure 9 is an exploded perspective view illustration of a kit-of-parts for assembling an aerosol delivery system 10.

As will be appreciated, in the arrangements described above, the fluid-transfer article 34 is provided within a housing 32 of the aerosol carrier 14. In such arrangements, the housing of the carrier 14 serves to protect the aerosol precursor-containing fluid-transfer article 34, whilst also allowing the carrier 14 to be handled by a user without his/her fingers coming into contact with the aerosol precursor liquid retained therein.

In any of the embodiments described above the second plate 36b of the second region 36 may have a thickness of less than 5mm. In other embodiments it may have a thickness of: less than 3.5mm, less than 3mm, less than 2.5mm, less than 2mm, less than 1.9mm, less than 1.8mm, less than 1.7mm, less than 1.6mm, less than 1 .5mm, less than 1.4mm, less than 1.3mm, less than 1.2mm, less than 1.1mm, less than 1mm, less than 0.9mm, less than 0.8mm, less than 0.7mm, less than 0.6mm, less than 0.5mm, less than 0.4mm, less than 0.3mm, less than 0.2mm, or less than 0.1mm.

The features disclosed in the foregoing description, or in the following claims, or in the accompanying drawings, expressed in their specific forms or in terms of a means for performing the disclosed function, or a method or process for obtaining the disclosed results, as appropriate, may, separately, or in any combination of such features, be utilised for realising the invention in diverse forms thereof, as long as the resulting combination of features falls within the scope of the claims.

While the invention has been described in conjunction with the exemplary embodiments described above, many equivalent modifications and variations will be apparent to those skilled in the art when given this disclosure. Accordingly, the exemplary embodiments of the invention set forth above are considered to be illustrative and not limiting. Various changes to the described embodiments may be made without departing from the scope of the invention as defined by the claims.

For the avoidance of any doubt, any theoretical explanations provided herein are provided for the purposes of improving the understanding of a reader. The inventors do not wish to be bound by any of these theoretical explanations.

Any section headings used herein are for organizational purposes only and are not to be construed as limiting the subject matter described.

Throughout this specification, including the claims which follow, unless the context requires otherwise, the words "have", "comprise", and "include", and variations such as "having", "comprises", "comprising", and "including" will be understood to imply the inclusion of a stated integer or step or group of integers or steps but not the exclusion of any other integer or step or group of integers or steps.

It must be noted that, as used in the specification and the appended claims, the singular forms "a," "an," and "the" include plural referents unless the context clearly dictates otherwise. Ranges may be expressed herein as from "about" one particular value, and/or to "about" another particular value. When such a range is expressed, another embodiment includes from the one particular value and/or to the other particular value. Similarly, when values are expressed as approximations, by the use of the antecedent "about," it will be understood that the particular value forms another embodiment. The term "about" in relation to a numerical value is optional and means, for example, +/- 10%.

The words "preferred" and "preferably" are used herein refer to embodiments of the invention that may provide certain benefits under some circumstances. It is to be appreciated, however, that other embodiments may also be preferred under the same or different circumstances. The recitation of one or more preferred embodiments therefore does not mean or imply that other embodiments are not useful, and is not intended to exclude other embodiments from the scope of the invention as defined by the claims.

## Claims

1. An aerosol delivery system (10) comprising an aerosol-generation apparatus (12) comprising a heater (24) and a fluid-transfer article (34), the fluid-transfer article forming the consumable part of the apparatus, said fluid-transfer article (34) comprising a first region (35) for holding an aerosol precursor and for transferring said aerosol precursor to a second region (36) of said fluid-transfer article (34), said second region (36) comprising a first part (36a) of a first material, said first part (36a) being adjacent said first region (35) and having a plurality of holes (37) therein, and a second part (36b) of a second material different from the first material, said second part (36b) being adjacent to said first part (36a) and extending across said plurality of holes (37) in said first part (36a); wherein said plurality of holes (37) are sized so that they cooperate with said second part (36b) of said second region (36) to define non-capillary spaces in said second region (36) into which said aerosol precursor is able to flow from said first region (35) in a non-capillary manner, thereby to impinge on said second part (36b); wherein said second part (36b) of said second region (36) is porous for passage therethrough of said aerosol precursor from said plurality of holes (37) to an activation surface (41) of said second region (36); wherein said heater (24) contacts said activation surface (41) so as to interact thermally with said activation surface (41); and
wherein said heater (24) and said activation surface (41) are separable,
wherein said system (10) comprises a carrier (14) comprising a first housing (32) containing said fluid-transfer article (34) and said system (10) comprises a further housing (43) supporting said heater (24), said first housing (32) and said further housing (43) being mutually detachable to allow the carrier to be removed from the rest of the aerosol delivery system,
**characterised in that**:
said second material is resistant to higher temperatures than said first material.

2. An aerosol delivery system (10) according to claim 1, wherein said heater (24) is a coil, mesh or foil.

3. An aerosol delivery system (10) according to claim 1 or claim 2, the apparatus having an air-flow pathway adjacent at least a part of said activation surface (41).

4. An aerosol delivery system (10) according to claim 3, wherein a part of said air-flow pathway is on the opposite side of said heater (24) from said activation surface (41).

5. An aerosol delivery system (10) according to any one of the preceding claims, wherein said first part (36a) of said second region (36) is formed of a solid polymer material having said plurality of holes (37) therein.

6. An aerosol delivery system (10) according to any one of the preceding claims, wherein said second part (36b) of said second region (36) is formed of fibrous material.

7. An aerosol delivery system (10) according to claim 6, wherein said fibrous material is ceramic fibre, glass fibre or carbon fibre.

8. An aerosol delivery system (10) according to any one of claims 1 to 5, where said second part (36b) of said second region (36) is formed from porous glass or porous ceramic material.

9. An aerosol delivery system (10) according to any one of claims 1 to 5, wherein said second part (36b) of said second region (36) is formed of a porous polymer material.

10. An aerosol delivery system (10) according to any one of the preceding claims, wherein said first region (35) of said fluid-transfer article (34) is of porous polymer material.

11. An aerosol delivery system (10) according to any one of claims 1 to 9, wherein said first region of said fluid-transfer article is a tank defining a hollow reservoir for the receipt of aerosol precursor.

12. An aerosol delivery system (10) according to any one of the preceding claims, wherein said plurality of holes (37) are moulded holes.

13. An aerosol delivery system (10) according to claim 3 or according to any one of claims 4 to 12 as dependent on claim 3, wherein said further housing (43) has an inlet (50) and said air-flow pathway extends to said inlet (50).

14. An aerosol delivery system (10) according to claim 13, wherein said further housing (43) has a plate (33) spaced from said heater (24) and said activation surface (41), so that said air-flow pathway passes between said plate (33) and said activation surface (41).

15. An aerosol delivery system (10) according to claim 14, wherein said plate (33) has a plurality of recesses (31) in its surface facing the activation surface (41), the air-flow pathway passing through said recesses (31).

## Patentansprüche

1. Aerosolzufuhrsystem (10), umfassend eine Aerosolerzeugungsvorrichtung (12), umfassend ein Heizelement (24) und einen Fluidtransportartikel (34), wobei der Fluidtransportartikel den konsumierbaren Teil der Vorrichtung bildet, wobei der Fluidtransportartikel (34) einen ersten Bereich (35) zum Enthalten eines Aerosolvorläufers und zum Zuführen des Aerosolvorläufers zu einem zweiten Bereich (36) des Fluidtransportartikels (34) umfasst, wobei der zweite Bereich (36) einen ersten Teil (36a) aus einem ersten Material, wobei der erste Teil (36a) benachbart zu dem ersten Bereich (35) angeordnet ist und eine Vielzahl von Löchern (37) darin aufweist, und einen zweiten Teil (36b) aus einem zweiten Material, das sich von dem ersten Material unterscheidet, umfasst, wobei der zweite Teil (36b) benachbart zu dem ersten Teil (36a) angeordnet ist und sich über die Vielzahl von Löchern (37) in dem ersten Teil (36a) erstreckt; wobei die Vielzahl von Löchern (37) so bemessen ist, dass sie mit dem zweiten Teil (36b) des zweiten Bereichs (36) zusammenwirkt, um nichtkapillare Räume in dem zweiten Bereich (36) zu definieren, in die der Aerosolvorläufer aus dem ersten Bereich (35) auf nichtkapillare Weise strömen kann, wodurch er auf den zweiten Teil (36b) auftrifft; wobei der zweite Teil (36b) des zweiten Bereichs (36) für das Durchströmen des Aerosolvorläufers aus der Vielzahl von Löchern (37) zu einer Aktivierungsoberfläche (41) des zweiten Bereichs (36) porös ist; wobei das Heizelement (24) die Aktivierungsoberfläche (41) berührt, um thermisch mit der Aktivierungsoberfläche (41) wechselzuwirken; und
wobei das Heizelement (24) und die Aktivierungsoberfläche (41) trennbar sind, wobei das System (10) einen Träger (14) umfasst, der ein erstes Gehäuse (32), das den Fluidtransportartikel (34) enthält, und das System (10) ein weiteres Gehäuse (43) umfasst, das das Heizelement (24) trägt, wobei das erste Gehäuse (32) und das weitere Gehäuse (43) gegenseitig abnehmbar sind, um zu ermöglichen, dass der Träger vom Rest des Aerosolzufuhrsystems entfernt werden kann, **dadurch gekennzeichnet, dass**:
das zweite Material gegenüber höheren Temperaturen als das erste Material beständig ist.

2. Aerosolzufuhrsystem (10) nach Anspruch 1, wobei das Heizelement (24) eine Spule, ein Gitter oder eine Folie ist.

3. Aerosolzufuhrsystem (10) nach Anspruch 1 oder Anspruch 2, wobei die Vorrichtung einen Luftströmungsweg benachbart zu zumindest einem Teil der Aktivierungsoberfläche (41) umfasst.

4. Aerosolzufuhrsystem (10) nach Anspruch 3, wobei ein Teil des Luftströmungswegs auf der dem Heizelement (24) entgegengesetzten Seite von der Aktivierungsoberfläche (41) angeordnet ist.

5. Aerosolzufuhrsystem (10) nach einem der vorangegangenen Ansprüche, wobei der erste Teil (36a) des zweiten Bereichs (36) aus einem festen Polymermaterial ausgebildet ist, das die Vielzahl von Löchern (37) darin ausgebildet aufweist.

6. Aerosolzufuhrsystem (10) nach einem der vorangegangenen Ansprüche, wobei der zweite Teil (36b) des zweiten Bereichs (36) aus faserartigem Material ausgebildet ist.

7. Aerosolzufuhrsystem (10) nach Anspruch 6, wobei das faserartige Material Keramikfaser, Glasfaser oder Kohlenstofffaser ist.

8. Aerosolzufuhrsystem (10) nach einem der Ansprüche 1 bis 5, wobei der zweite Teil (36b) des zweiten Bereichs (36) aus porösem Glas oder porösem Keramikmaterial ausgebildet ist.

9. Aerosolzufuhrsystem (10) nach einem der Ansprüche 1 bis 5, wobei der zweite Teil (36b) des zweiten Bereichs (36) aus einem porösen Polymermaterial ausgebildet ist.

10. Aerosolzufuhrsystem (10) nach einem der vorangegangenen Ansprüche, wobei der erste Bereich (35) des Fluidtransportartikels (34) aus einem porösen Polymermaterial ausgebildet ist.

11. Aerosolzufuhrsystem (10) nach einem der Ansprüche 1 bis 9, wobei der erste Bereich des Fluidtransportartikels ein Tank ist, der ein Hohlraumreservoir für die Aufnahme des Aerosolvorläufers definiert.

12. Aerosolzufuhrsystem (10) nach einem der vorangegangenen Ansprüche, wobei die Vielzahl von Löchern (37) geformte Löcher sind.

13. Aerosolzufuhrsystem (10) nach Anspruch 3 oder nach einem der Ansprüche 4 bis 12 in Abhängigkeit von Anspruch 3, wobei das weitere Gehäuse (43) einen Einlass (50) aufweist und wobei sich der Luftströmungsweg bis zu dem Einlass (50) erstreckt.

14. Aerosolzufuhrsystem (10) nach Anspruch 13, wobei das weitere Gehäuse (43) eine Platte (33) aufweist, die von dem Heizelement (24) und der Aktivierungsoberfläche (41) beabstandet ist, sodass der Luftströmungsweg zwischen der Platte (33) und der Aktivierungsoberfläche (41) hindurchströmt.

15. Aerosolzufuhrsystem (10) nach Anspruch 14, wobei die Platte (33) eine Vielzahl von Vertiefungen (31) in deren Oberfläche aufweist, die der Aktivierungsoberfläche (41) zugewandt sind, wobei der Luftströmungsweg durch die Vertiefungen (31) hindurchführt.

## Revendications

1. Système de distribution d'aérosol (10) comprenant un appareil de génération d'aérosol (12) comprenant un dispositif de chauffage (24) et un article de transfert de fluide (34), l'article de transfert de fluide formant la partie consommable de l'appareil, ledit article de transfert de fluide (34) comprenant une première région (35) pour contenir un précurseur d'aérosol et pour transférer ledit précurseur d'aérosol vers une seconde région (36) dudit article de transfert de fluide (34), ladite seconde région (36) comprenant une première partie (36a) d'un premier matériau, ladite première partie (36a) étant adjacente à ladite première région (35) et présentant une pluralité de trous (37) en son sein, et une seconde partie (36b) d'un second matériau différent du premier matériau, ladite seconde partie (36b) étant adjacente à ladite première partie (36a) et s'étendant à travers ladite pluralité de trous (37) dans ladite première partie (36a) ; dans lequel ladite pluralité de trous (37) sont dimensionnés de telle sorte qu'ils coopèrent avec ladite seconde partie (36b) de ladite seconde région (36) pour définir des espaces non capillaires dans ladite seconde région (36) dans lesquels ledit précurseur d'aérosol peut s'écouler à partir de ladite première région (35) d'une manière non capillaire, de manière à heurter ainsi ladite seconde partie (36b) ; dans lequel ladite seconde partie (36b) de ladite seconde région (36) est poreuse pour le passage à travers celle-ci dudit précurseur d'aérosol depuis ladite pluralité de trous (37) vers une surface d'activation (41) de ladite seconde région (36) ; dans lequel ledit dispositif de chauffage (24) vient en contact avec ladite surface d'activation (41) de manière à interagir thermiquement avec ladite surface d'activation (41) ; et
dans lequel ledit dispositif de chauffage (24) et ladite surface d'activation (41) peuvent être séparés,
dans lequel ledit système (10) comprend un support (14) comprenant un premier logement (32) contenant ledit article de transfert de fluide (34) et ledit système (10) comprend un logement supplémentaire (43) supportant ledit dispositif de chauffage (24), ledit premier logement (32) et ledit logement supplémentaire (43) pouvant être détachés mutuellement afin de permettre au support d'être retiré du reste du système de distribution d'aérosol,
**caractérisé en ce que** :
ledit second matériau résiste à des températures plus élevées que ledit premier matériau.

2. Système de distribution d'aérosol (10) selon la revendication 1, dans lequel ledit dispositif de chauffage (24) est une bobine, un treillis ou une feuille.

3. Système de distribution d'aérosol (10) selon la revendication 1 ou la revendication 2, l'appareil présentant un trajet d'écoulement d'air adjacent à au moins une partie de ladite surface d'activation (41).

4. Système de distribution d'aérosol (10) selon la revendication 3, dans lequel une partie dudit trajet d'écoulement d'air se trouve sur le côté opposé dudit dispositif de chauffage (24) par rapport à ladite surface d'activation (41).

5. Système de distribution d'aérosol (10) selon l'une quelconque des revendications précédentes, dans lequel ladite première partie (36a) de ladite seconde région (36) est formée en un matériau polymère solide présentant ladite pluralité de trous (37) en son sein.

6. Système de distribution d'aérosol (10) selon l'une quelconque des revendications précédentes, dans lequel ladite seconde partie (36b) de ladite seconde région (36) est formée en matériau fibreux.

7. Système de distribution d'aérosol (10) selon la revendication 6, dans lequel ledit matériau fibreux est de la fibre de céramique, de la fibre de verre ou de la fibre de carbone.

8. Système de distribution d'aérosol selon l'une quelconque des revendications 1 à 5, dans lequel ladite seconde partie (36b) de ladite seconde région (36) est formée à partir de verre poreux ou de matériau de céramique poreux.

9. Système de distribution d'aérosol (10) selon l'une quelconque des revendications 1 à 5, dans lequel ladite seconde partie (36b) de ladite seconde région (36) est formée d'un matériau polymère poreux.

10. Système de distribution d'aérosol (10) selon l'une quelconque des revendications précédentes, dans lequel ladite première région (35) dudit article de transfert de fluide (34) est en matériau polymère poreux.

11. Système de distribution d'aérosol (10) selon l'une quelconque des revendications 1 à 9, dans lequel ladite première région dudit article de transfert de fluide est une cuve définissant un réservoir creux pour la réception de précurseur d'aérosol.

12. Système de distribution d'aérosol (10) selon l'une quelconque des revendications précédentes, dans lequel ladite pluralité de trous (37) sont des trous moulés.

13. Système de distribution d'aérosol (10) selon la revendication 3 ou l'une quelconque des revendication 4 à 12 lorsqu'elle dépend de la revendication 3, dans lequel ledit logement supplémentaire (43) présente une entrée (50) et ledit trajet d'écoulement d'air s'étend vers ladite entrée (50).

14. Système de distribution d'aérosol (10) selon la revendication 13, dans lequel ledit logement supplémentaire (43) présente une plaque (33) espacée dudit dispositif de chauffage (24) et de ladite surface d'activation (41), de sorte que ledit trajet d'écoulement d'air passe entre ladite plaque (33) et ladite surface d'activation (41).

15. Système de distribution d'aérosol (10) selon la revendication 14, dans lequel ladite plaque (33) présente une pluralité d'évidements (31) dans sa surface faisant face à la surface d'activation (41), le trajet d'écoulement d'air passant à travers lesdits évidements (31).
